# EUROPÄISCHE PATENTANMELDUNG

(11) **EP 1 013 265 A2**
(43) Veröffentlichungstag der Anmeldung: **28.06.2000**
(21) Anmeldenummer: 99125656.1
(22) Anmeldetag: 22.12.1999
(51) Int. Cl.: A61K 7/48

(54) **Disperses System als Hautpflegemittel**

(30) Priorität: 22.12.1998 DE 19859455
(71) Anmelder: DIANORM G. Maierhofer GmbH, 81247 München (DE)
(72) Erfinder: Maierhofer,Günther, Dianorm G.Maierhofer GmbH, 81247 München (DE)
(74) Vertreter: Grünecker, Kinkeldey, Stockmair & Schwanhäusser Anwaltssozietät

(57) **Zusammenfassung**

Die Erfindung betrifft ein disperses System und seine Verwendung als Hautpflegemittel, sowie ein Verfahren zum Herstellen des dispersen Systems. Aufgabe der Erfindung ist es, ein Hautpflegemittel bereitzustellen, das der Haut vor allem Wasser und Lipide aktiv zuführt und schnell in die Haut eindringt, ohne auf der Hautoberfläche eine Fettschicht zu hinterlassen. Diese Aufgabe wird erfindungsgemäß durch ein disperses System gelöst, das Wasser und ein oder mehrere Phospholipide enthält und eine Viskosität von 1-15 mPa.s aufweist. In einer bevorzugten Ausführungsform der Erfindung sind die Phospholipide Lecithine, und insbesondere ein Rohlecithin pflanzlichen Ursprungs wie Sojalecithin. Das erfindungsgemäße disperse System liegt vorzugsweise in Form eines wäßrigen Nanokolloids mit einem Teilchendurchmesser von kleiner als 250 nm, insbesondere kleiner als 150 nm, vor.

## Beschreibung

Die Erfindung betrifft ein disperses System und seine Verwendung als Hautpflegemittel, sowie ein Verfahren zur Herstellung des dispersen Systems.

Die Haut ist ein Iebensnotwendiges Organ des menschlichen Körpers, das als Schutzbarriere gegen Austrocknung und gegen äußere mechanische, chemische und thermische Einwirkungen dient. Die Haut unserer Hände wird durch äußere Einflüsse am stärksten strapaziert. Schädigungen werden z.B. durch Arbeiten mit Lösungsmitteln, Säuren, Laugen, Schmiermitteln oder Ölen, durch trockene Luft, Kälte oder Hitze, durch Handwaschpasten oder auch nur durch häufiges Waschen mit Seifen hervorgerufen. Betroffen hiervon ist die Mehrzahl der Bevölkerung, ganz besonders aber bestimmte Berufsgruppen wie z.B. Laborpersonal, Klinikpersonal, Ärzte, Friseure, viele Handwerker sowie im Haushalt beschäftigte Personen. Die Haut verliert Wasser, Lipide und den Säureschutzmantel und leidet unter vielen kleinen bis winzigen Verletzungen, unter anderem unter Hautrissen wie sie bei Handwerkern, z.B. Kfz-Schlossern, Schreinern oder Fliesenlegern auftreten. Auch ohne solche extremen Einflüsse verliert die Haut im Laufe der Jahre Wasser, Lipide und Spannkraft, was zu der sog. Altershaut führt.

Hautpflegemittel erfüllen vor allem den Zweck, den Fett- und Feuchtigkeitsverlust der Haut infolge von Waschen, Witterungs- und Umwelteinflüssen usw. auszugleichen und die Haut gegen Austrocknung zu schützen. Zu diesem Zweck gibt es auf dem Markt eine Vielzahl von Präparaten, bei denen es sich vor allem um Cremes handelt, jedoch werden auch Gele, Öle und Lotios angeboten. Bekannte Vertreter sind u.a. Nivea®, Bebe®, Penaten®, Atrix®, oder Handsan®. Allen diesen Präparaten gemeinsam ist eine hohe bis sehr hohe Viskosität, eine sehr geringe Penetration in die Haut, dem Wirkort, und eine Vielzahl von Inhaltsstoffen. So gibt z.B. die Firma Beiersdorf für die Nivea® Creme folgende Inhaltsstoffe an: Aqua, Paraffinum liquidum, Cera microcristallina, Glycerin, Lanolin, Alkohol, Paraffin, Ceresin, Magnesiumsulfat, Decyloleat, Octyldodecanol, Aluminiumstearat, Panthenol, Zitronensäure, Parfüm.

Ein ideales Hautpflegemittel muß der Haut vor allem Wasser und Lipide aktiv zuführen und tief genug in die Haut eindringen. Die im Handel erhältlichen Hautpflegemittel erfüllen trotz zahlreicher Bestandteile und Wirkstoffe diese Forderung allesamt nicht zufriedenstellend.

Öle machen die Haut zwar geschmeidig, können ihr aber kein Wasser zuführen. Sie wirken eher als isolierende und verdunstungshemmende Schutzschicht, eine tiefe Penetration in die Haut findet jedoch nicht statt. Öle enthalten auch kaum Lipide, die die Haut verwerten kann.

Gele, insbesondere Hydrogele, enthalten zwar Wasser, aber keine Lipide. Dar-über hinaus ist eine Penetration der Inhaltsstoffe in die Haut durch das Gelgerüst nahezu unmöglich. Oft verbleiben nach dem Auftragen und Austrocknen regelrechte, mit dem Finger wieder abziehbare Filme auf der Haut zurück. Ein anschauliches Beispiel stellt das Gelpräparat Voltaren-Gel® dar, das von Orthopäden zur Behandlung schmerzhafter Prellungen und Knieverletzungen verwendet wird. Das Präparat besteht aus einem Gel und dem Entzündungshemmer und Analgetikum Diclofenac. Soll der Wirkstoff seine Wirkung entfalten, so muß er tief in die Haut eindringen und zwar bis in die blutversorgte Dermis. Da dies weder durch einfaches Auftragen des Gels auf die Haut oder durch Massage mit den Fingern gelingt, ist sogar schon versucht worden, die Haut mittels Ultraschall oder galvanischem Strom permeabler zu machen, so daß wenigstens ein kleiner Teil des Wirkstoffs die Dermis und damit die lebenden Zellen erreicht.

Cremes (mit wenig Wasser) und Lotios (mit etwas mehr Wasser) erfüllen die vorstehend erwähnten Anforderungen ebenfalls nicht. Cremes überziehen die Haut mit einer mehr oder weniger homogenen, unterschiedlich starken und glänzenden Fettschicht, die in den obersten Hautschichten (Epidermis) bleibt und Hautrisse glättet. Obwohl sie die Haut geschmeidig machen, können sie ihr weder Wasser noch hautverwertbare Lipide aktiv zuführen, da sie die Lipide nicht in tiefere Hautschichten transportieren und dort bioverfügbar machen können. Der beobachtbare Pflegeeffekt kommt dadurch zustande, daß Cremes die Wasserverdunstung verzögern.

Neben den positiven Eigenschaften von Cremes bestehen aber gravierende Nachteile. Soll die Creme gut in die Haut einziehen, muß sie minutenlang einmassiert werden. Nach dem Einmassieren ist die Haut jedoch fettig, manchmal auch klebrig und es dauert oft bis zu einer halben Stunde, bis sich die Haut wieder trocken anfühlt. Selbst nach dieser Zeit hinterlassen eingecremte Finger deutliche Fettspuren auf z.B. Gläsern; außerdem besteht beim Arbeiten mit glatten Werkzeugen wie z.B. Schraubenschlüsseln Abgleitgefahr. Aus diesen Gründen kommen Cremes für den täglichen Gebrauch während der Arbeitszeit überhaupt nicht in Betracht, obwohl eine tägliche Pflege aus medizinischer Sicht sinnvoll ist und auch ein Bedürfnis danach besteht.

Es ist deshalb Aufgabe der Erfindung, ein Hautpflegemittel bereitzustellen, das die vorstehend genannten Nachteile von Ölen, Gelen und Cremes überwindet.

Diese Aufgabe wird erfindungsgemäß durch ein disperses System gelöst, das Wasser und ein oder mehrere Phospholipide enthält und eine Viskosität von 1-15 mPa.s aufweist. Vorzugsweise weist das erfindungsgemäße disperse System eine Viskosität von 1-10 mPa.s auf, und besonders bevorzugt ist ein disperses System mit einer Viskosität von 1-5 mPa.s.

Das disperse System gemäß der Erfindung führt der Haut nach dem Auftragen Wasser und hautverwertbare Phospholipide aktiv zu und macht diese Verbindungen bioverfügbar. Dadurch wird die Haut sichtbar und spürbar glatt und samtig. Schuppen, rauhe Haut und Schwielen verschwinden, Hautrisse heilen rascher ab. Die natürliche Schutzfunktion der Haut wird wiederhergestellt bzw. erhalten.

Im Gegensatz zu den im Handel erhältlichen Hautpflegepräparaten ist die Viskosität des dispersen Systems gemäß der Erfindung äußerst gering. Zum Vergleich: Monosubstanzen wie z.B. Glycerin und Rhizinusöl haben Viskositäten von 1600 bzw. 1060, dünnflüssige Lotios bereits zwischen 600 und 1000 mPa.s. Wegen seiner niedrigen Viskosität dringt das erfindungsgemäße disperse System innerhalb von Sekunden tief in die Haut ein. Die Haut fühlt sich sofort wieder trocken an; sie wird weder fettig noch klebrig und die Finger hinterlassen keinerlei Spuren auf glatten Gegenständen. Das erfindungsgemäße disperse System kann als Hautpflegemittel nach jeder Händereinigung angewendet werden, die lästige und hinderliche "Wartezeit" wie bei Cremes entfällt und der Benutzer kann praktisch sofort weiterarbeiten. Außerdem ist der Verbrauch, verglichen mit Cremes, sehr viel geringer, da für eine vergleichbare Wirkung sehr viel weniger Mittel benötigt wird.

Besonders vorteilhaft ist das disperse System gemäß der Erfindung bei seiner Anwendung im medizinischen Bereich, beispielsweise in Krankenhäusern oder Arztpraxen. Wegen seiner geringen Viskosität kann das disperse System problemlos mit Hilfe von Sprühern und Dispensern dosiert werden, wodurch eine etwaige Verunreinigung mit Schmutz oder eine Kontamination mit Krankheitserregern vermieden wird. Es ist ebenfalls möglich das erfindungsgemäße disperse System durch Filtration zu sterilisieren. Man erhält dann ein steriles Hautpflegemittel, das völlig frei von Schwebstoffen und Partikeln ist.

In einer bevorzugten Ausführungsform des erfindungsgemäßen dispersen Systems sind als Phospholipid ein oder mehrere Lecithine enthalten. Lecithine, auch Phosphatidylcholine genannt, bilden sich durch Veresterung von Fettsäuren, Glycerin, Phosphorsäure und Cholin. Besonders reichlich kommen sie in ölhaltigen Pflanzensamen (z.B. Rapssamen), Sojabohnen, Getreide und Erdnüssen, aber auch im Herzmuskel, Blutplasma, in Hirn- und Nervengewebe, Sperma, Eigelb und Hefen vor. Lecithine sind Bestandteile der Zellmembranen aller Lebewesen. Als Phospholipide sind sie hautverwertbar und können in verletzte Zellmembranen eingebaut werden.

Vorzugsweise enthält das erfindungsgemäße disperse System Rohlecithin pflanzlichen Ursprungs. Beispiele für Rohlecithine pflanzlichen Ursprungs sind die bereits genannten Lecithine aus Rapssamen, Getreide oder Erdnüssen. Besonders bevorzugt ist das aus Sojabohnen extrahierte Lecithin, das ein Gemisch aus verschiedenen Lecithinen mit unterschiedlichen Fettsäure-Resten, z.B. Palmitinsäure, Stearinsäure, Palmitoleinsäure, Oleinsäure, Linolsäure und Linolensäure, enthält. Das disperse System gemäß der Erfindung kann auch entöltes, mit Phosphatidylcholin angereichertes Sojalecithin enthalten. Das Lecithin kann in einer Konzentration von 0,1-1,0 Gew.-% vorliegen, vorzugsweise 0,3 Gew.-%.

In einer weiteren Ausführungsform der Erfindung enthält das disperse System neben Wasser und Phospholipiden eine rückfettende Substanz. Als rückfettende Substanz kann ein Öl eingesetzt werden, vorzugsweise ein Neutralöl, besonders bevorzugt Miglyol. Die rückfettende Substanz kann beispielsweise in einer Konzentration von 0,01-0,3 Gew.-% vorliegen; bevorzugt ist eine Konzentration der rückfettenden Substanz im dispersen System von 0,05 Gew.-%.

Ferner kann das erfindungsgemäße disperse System mindestens ein Antioxidans, vorzugsweise Vitamin A-Palmitat oder Tocopherolacetat enthalten. Das Antioxidans wirkt als Radikalfänger und verhindert auch das Ranzigwerden der Phospholipide bei der Lagerung. Beispielsweise kann das Antioxidans in einer Konzentration von 0,001-0,015 Gew.-% vorliegen, bevorzugt ist eine Konzentration von 0,0075 Gew.-%.

Um die Lagerfähigkeit des erfindungsgemäßen dispersen Systems zu verbessern, kann dieses ein Konservierungsmittel enthalten, vorzugsweise 2-Phenoxy-ethanol oder Chlorhexidin. Ferner können auch analgetisch wirkende Substanzen wie z.B. Nelkenöl, wundheilungsfördernde Substanzen wie z.B. Dexpanthenol oder antiseptisch wirkende Substanzen wie z.B. Teebaumöl oder Chlorhexidindiacetat enthalten sein.

In einer besonders bevorzugten Ausführungsform, der Erfindung liegt das erfindungsgemäße disperse System in Form eines wäßrigen Nanokolloids mit einem Teilchendurchmesser von kleiner als 250 nm, vorzugsweise kleiner als 150 nm vor.

Die durch Dispergierung von Phospholipiden, z.B. von Lecithinen, in Wasser entstehenden Teilchen sind kugelförmige, mehr oder weniger homogene, mit Wasser gefüllte Vesikel, deren "Außenhaut" (Membran) aus Lecithin besteht und die im Wasser schweben. Die Größe der Vesikel ist abhängig vom Energieeintrag bei der Herstellung. Sie penetrieren rasch und tief in die Haut und führen ihr lebensnotwendige "Nährsubstanzen" wie die hautverwertbaren Phospholipide und Wasser aktiv zu; auch andere im dispersen System enthaltene Substanzen, beispielsweise Kohlenhydrate (Zucker) oder Glykolipide, können dadurch der Haut zugeführt werden. Sofern die dispergierten Phospholipide natürlichen Ursprungs sind, wie im Falle natürlicher Lecithine, sind die Vesikel physiologisch unbedenklich und haben kein allergisches Potential.

Das erfindungsgemäße disperse System kann als Hautpflegemittel verwendet werden oder die Grundlage eines Hautpflegemittels bilden.

Die Erfindung betrifft ferner die Herstellung eines dispersen Systems wie vorstehend beschrieben, beispielsweise durch Dispergieren der Inhaltsstoffe in Wasser mittels eines Dispergiergerätes. Handelsübliche Dispergiergeräte wie z.B. Polytron, Ultra Turrax, Stephan UMC können dazu verwendet werden.

Das so hergestellte disperse System ist langzeitstabil (über 2 Jahre), kann auf einfache Weise in industriellen Mengen hergestellt werden und steril oder konserviert abgefüllt werden. Bei Verwendung als Kosmetikum stellt dieses System ein ideales Hautpflegemittel dar, insbesondere für die Haut der Hände, und kann aufgrund seiner Eigenschaften auch täglich während der Arbeitszeit angewandt werden.

### Beispiele

### Beispiel 1

### Herstellung von 240 kg dispersem System

0,72 kg Sojalecithin werden mit 5,28 kg destilliertem Wasser gemischt und mit Hilfe eines Dispergiergerätes (Stephan UMC 12) unter Vakuum 60 Minuten bei 3000 U/min dispergiert. Während des Dispergiervorgangs wird die Temperatur des Gemisches mittels Wasserkühlung bei Raumtemperatur konstant gehalten. Zum Erhalt eines dispersen Systems gemäß der Erfindung wird die erhaltene Dispersion mit 234 kg konserviertem Wasser (beispielsweise mit Chlorhexidin konserviertes Wasser) verdünnt.

Die mit Hilfe eines Oszillazionsviskosimeters (Visconorm, Gel Instrumente AG, Thalwil, Schweiz) gemessene Viskosität des erhaltenen dispersen Systems beträgt etwa 1 mPa.s. Der Teilchendurchmesser der nanokolloidalen Dispersion liegt bei etwa 150 nm.

### Beispiel 2

### Herstellung von 40 kg dispersem System mit Neutralöl

0,72 kg Sojalecithin und 0,12 kg Neutralöl, z.B. Miglyol 810, werden in 5,16 kg destilliertem Wasser wie in Beispiel 1 dispergiert und anschließend mit 233,88 kg konserviertem Wasser verdünnt. Die Viskosität des dispersen Systems beträgt etwa 1 mPa.s. Der Teilchendurchmesser der nanokolloidalen Dispersion liegt bei etwa 150 nm.

### Beispiel 3

### Herstellung von 240 kg dispersem System mit einem Neutralöl und einem Antioxidans

0,72 kg Sojalecithin, 0,12 kg Neutralöl, z.B. Miglyol 810, und 0,018 kg Antioxidans, z.B. Vitamin A-Palmitat, werden in 5,14 kg destilliertem Wasser wie in Beispiel 1 dispergiert und anschließend mit 233,8 kg konserviertem Wasser verdünnt. Die Viskosität des dispersen Systems beträgt etwa 1 mPa.s und der Teilchendurchmesser der nanokolloidalen Dispersion liegt bei etwa 150 nm.

Anstelle der Verdünnung mit konserviertem Wasser kann hierzu auch nicht konserviertes destilliertes Wasser benutzt werden. In diesem Fall wird das disperse System anschließend sterilfiltriert und steril in keimfreie Behälter abgefüllt.

## Patentansprüche

1. Disperses System, das Wasser und ein oder mehrere Phospholipide enthält und eine Viskosität von 1-15 mPa.s aufweist.

2. Disperses System nach Anspruch 1, **dadurch gekennzeichnet**, daß es sich bei dem/den Phospholipid/den Phospholipiden um Lecithin/Lecithine handelt.

3. Disperses System nach Anspruch 2, **dadurch gekennzeichnet**, daß mindestens ein Lecithin Rohlecithin pflanzlichen Ursprungs, vorzugsweise Sojalecithin, ist.

4. Disperses System nach Anspruch 2 oder 3, **dadurch gekennzeichnet**, daß das Lecithin entöltes, mit Phosphatidylcholin angereichertes Sojalecithin ist.

5. Disperses System nach einem der Ansprüche 2 bis 4, **dadurch gekennzeichnet**, daß Lecithin in einer Konzentration von 0,1-1,0 Gew.-% vorliegt, vorzugsweise 0,3 Gew.-%.

6. Disperses System nach einem der Ansprüche 1 bis 5, **dadurch gekennzeichnet**, daß eine rückfettende Substanz enthalten ist.

7. Disperses System nach Anspruch 6, **dadurch gekennzeichnet**, daß die rückfettende Substanz ein Öl ist, vorzugsweise ein Neutralöl, besonders bevorzugt Miglyol.

8. Disperses System nach Anspruch 6 oder 7, **dadurch gekennzeichnet**, daß die rückfettende Substanz in einer Konzentration von 0,01-0,3 Gew.-% vorliegt, vorzugsweise 0,05 Gew.-%.

9. Disperses System nach einem der Ansprüche 1 bis 8, **dadurch gekennzeichnet**, daß mindestens ein Antioxidans, vorzugsweise Vitamin A-Palmitat oder Tocopherolacetat, enthalten ist.

10. Disperses System nach Anspruch 9, **dadurch gekennzeichnet**, daß das Antioxidans in einer Konzentration von 0,001-0,015 Gew.-% vorliegt, vorzugsweise 0,0075 Gew.-%.

11. Disperses System nach einem der Ansprüche 1 bis 10, **dadurch gekennzeichnet**, daß mindestens ein Konservierungsmittel, vorzugsweise 2-Phenoxy-ethanol oder Chlorhexidin, enthalten ist.

12. Disperses System nach einem der vorstehenden Ansprüche in Form eines wäßrigen Nanokolloids mit einem Teilchendurchmesser von kleiner als 250 nm, vorzugsweise kleiner als 150 nm.

13. Verwendung des dispersen Systems nach einem der Ansprüche 1 bis 12 als Hautpflegemittel.

14. Verfahren zum Herstellen eines dispersen Systems nach einem der Ansprüche 1 bis 12 durch Dispergieren der Inhaltsstoffe in Wasser mittels eines Dispergiergerätes.
